# EUROPEAN PATENT SPECIFICATION

(11) **EP 0 595 197 B1**
(45) Date of publication and mention of the grant of the patent: **02.04.1997**
(21) Application number: 93117051.8
(22) Date of filing: 21.10.1993
(51) Int. Cl.: C07C 43/168, C11B 9/00

(54) **Enol ethers and odorant compositions containing them**
Enolether und sie enthaltende Riechstoffkompositionen
Ethers d'enols et compositions odorantes les contenant

(30) Priority: 26.10.1992 EP 92810824
(43) Date of publication of application: 04.05.1994
(73) Proprietor: GIVAUDAN-ROURE (INTERNATIONAL) S.A., CH-1214 Vernier, Genève (CH)
(72) Inventor: Baudin, Josianne, F-74100 Annemasse (FR)
(74) Representative: Urech, Peter, Dr.

(56) References cited:
- EP-A- 0 379 981
- US-A- 4 250 200
- US-A- 4 650 603

## Description

The invention relates to novel odorants, namely enol ethers of alkyl-substituted oxo-tetralins and oxo-indanes, for example of oxo-tetralins and oxo-indanes having up to 6 or 7 alkyl substituents.

In particular, the invention is concerned with the compounds of the formula 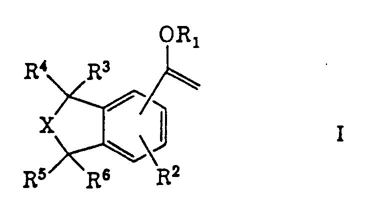 wherein
R¹ is C₁₋₄ alkyl or C₂₋₄ alkenyl
R² signifies H or C₁₋₄ alkyl
R³, R⁴, R⁵, R⁶ signify H, CH₃, C₂H₅, CH₂-CH₂-CH₃ or CH(CH₃)₂,
X is methylene, ethylidene, propylidene, ethylene, propylene, isopropylidene or 1,2-dimethylethylene,
and the total number of carbon atoms of R³, R⁴, R⁵, R⁶ is ≤ 6.

The compounds of formula I are distinguished by very natural notes in the direction of musk, woody, sweet, fruity, floral.

An important technical advantage in the use of the enol ethers I results from the fact that - this in contrast to the corresponding ketals II (see below) - they furnish liquid mixtures when blended with one or more Polycyclic Musks, (see e.g. Ernst T. Theimer in Fragrance Chemistry, The science of the Sense of Smell, pages 514 to 528 (1982)), describing such Polycyclic Musks, e.g. Fixolide, Crysolide, Phantolid, Novalide, Galaxolide®, etc.). A preferred execution is the blending with the parent carbonyl compounds III (see below).

Thus, it is possible by mixing the novel compounds I and known musk odorants, i.e. the corresponding basic carbonyl compounds III, such as Fixolide, Phantolide, Crysolide etc. to manufacture liquid musks of variable intensity. These mixtures are generally sweeter (generally with a fruity side note), better balanced and more natural than the Polycyclic Musks as such.

The optimal ratio of compounds I/Polycyclic Musks is in each case based on economic reasons according to the intended utilization (e.g. alcoholic perfumery or the perfuming of laundry, etc.) and of the desired fluidity of the mixtures obtained; this pronounced and varying fluidity of the mixtures of the carbonyl compounds III/enol ethers I being a further technical advantage. The ratio lies, for example, between about ca. 1:10 to ca. 1:1. For economic reasons the proportion of the more expensive enol ether is usually kept on the lower side.

The compounds I may be obtained by the elimination of an equivalent of the alcohol R¹OH from the ketal 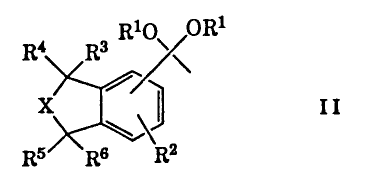 conveniently in the usual manner, expediently at elevated temperatures and acid-catalyzed.

Suitable acids are mineral acids, such as HCl, H₃PO₄, H₂SO₄, or H₃BO₃, acidic salts such as KHSO₄, organic acids such as p-toluene sulphonic acid, acidic ion exchangers, e.g. of the Amberlyst 15 type.

A suitable temperature range is between ca. 40°C and 120°C, preferably between ca. 60°C and ca. 80°C.

Solvents are optional, and are preferably selected from the group of alcanols and aliphatic or aromatic hydrocarbons.

The isolation is preferably effected by distillation under reduced pressure and column chromatography.

In case of the novel mixtures of carbonyls III and enol ethers I outlined above, it is convenient to prepare the enol ethers I from the compounds II prepared in situ.

According to EP-A₁ 379 981 the ketals II can be manufactured by reacting the basic ketone, i.e. the compound of formula 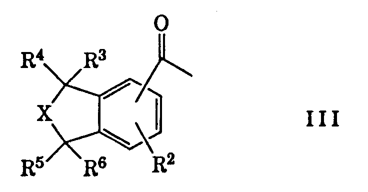 with an excess, e.g. a two fold excess of ortho formic esters in alcohols, and especially at temperatures between ca. 20° and ca. 40° C.

Scaling down the amount of ortho formic ester to below, say equimolar amounts, and raising the temperature above ca. 40° C, e.g. up to a maximum of ca. 120° C, leads directly to any desired mixtures of the ketones III and enol ethers I, because the ketal formed initially is this way immediately transferred to the compound I, thus leaving the "basic" ketone and novel compound I in the reaction vessel.

The parameters of this reaction are analogous to the ones outlined above.

The novel products of this invention (compounds I, compound I + Polycyclic Musks) combine with numerous known odorant ingredients of natural or synthetic origin, whereby the range of the natural raw substances can embrace not only readily-volatile, but also moderately-volatile and slightly-volatile components and that of the synthetics can embrace representatives from practically all classes of substances, as will be evident from the following compilation:
- natural products, such as tree moss absolute, basil oil, citrus fruit oils (such as bergamot oil, mandarin oil, etc.), mastix absolute, myrtle oil, palmarosa oil, patchouli oil, petitgrain oil Paraguay, wormwood oil,
- alcohols, such as farnesol, geraniol, linalool, nerol, phenylethyl acohol, rhodinol, cinnamic alcohol,
- aldehydes, such as citral, Helional®, α-hexyl-cinnamaldehyde, hydroxycitronellal, Lilial® (p-tert.butyl-α-methyl-dihydrocinnamaldehyde), methylnonylacetaldehyde,
- ketones, such as allylionone, α-ionone, β-ionone, isoraldein (isomethyl-α-ionone), methylionone,
- esters, such as allyl phenoxyacetate, benzyl salicylate, cinnamyl propionate, citronellyl acetate, citronellyl ethoxolate (citronellyl . O - CO - CO . OC₂H₅), decyl acetate, dimethylbenzylcarbinyl acetate, dimethylbenzylcarbinyl butyrate, ethyl acetoacetate, ethyl acetylacetate, hexenyl isobutyrate, linalyl acetate, methyl dihydrojasmonate, styrallyl acetate, vetiveryl acetate, etc.,
- lactones, such as γ-undecalactone,
- various components often used in perfumery, such as musk ketone, indole, p-menthane-8-thiol-3-one, methyl-eugenol.

The novel products can be used in wide limits which can range in compositions, for example, from about 0.1 (detergents) - about 20% by weight (alcoholic solutions), without these values being, however, limiting values, as the experienced perfumer can also achieve effects with even lower concentrations or can synthesize novel complexes (i.e. compositions) with even higher amounts. The preferred concentrations range between about 1% and about 10%. The compositions manufactured with I can be used for all kinds of perfumed consumer goods (eau de cologne, eau de toilette, extracts, lotions, creams, shampoos, soaps, salves, powders, toothpastes, mouth washes, deodorants, detergents, fabric conditioners, tobacco, etc.).

The products can accordingly be used in the manufacture of compositions and - as the above compilation shows - using a wide range of known odorants or odorant mixtures. In the manufacture of such compositions the known odorants referred to above can be used according to methods known to the perfumer such as, e.g. from W.A. Poucher, Perfumes, Cosmetics and Soaps 2, 7th Edition, Chapman and Hall, London, 1974.

### Examples

### General methology

A mixture of the appropriate ketal e.g. (0.6 mol) in 200 ml of toluene is treated with 0.5 g of p-toluene salfonic acid and is boiled in a round-bottomed flask equipped with a Hahn head (a dephlegmator device) and a vertical condenser. The alcohol R¹OH of the particular ketal is used as the cooling liquid in the Hahn head. The alcohol formed slowly in the reaction distills off and is collected in a measuring cylinder so that the progress of the reaction can easily be followed. After the alcohol has been eliminated, the reaction mass is neutralized by the addition of a 10% solution of potassium hydroxide in ethanol and the solvent is evaporated under reduced pressure. The pure enol ether is obtained by distillation under vacuum.

### Example 1

The following enol ethers have been prepared from the corresponding ketals according to the method outlined above.

### a) 6-(1-Ethoxy-vinyl)-1,1,4,4-tetramethyl-tetralin

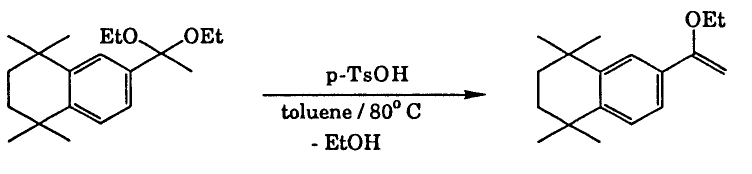

The distillation under vacuum of the crude product provides a colorless liquid enol ether which contains about 10% of the carbonyl compound III; bp: 150°C (2 mbar).
GC: column 30 meters x 0.53 mm, Carbowax stationary phase (Supelcowax) programmed at 120-220°C at 5°C per minute.
¹H-NMR (200 MHz; CDCl₃): 1.27 (s,6H); 1.29 (s,6H), 1.42 (t,J=7Hz,3H); 1.68 (s,4H); 3.91 (q,J=7Hz,2H), 4.14 (d,J=2Hz,1H); 4.57 (d,J=2Hz,1H); 7.24 at 7.40 (m,2H); 7.56 (d,J=2Hz,1H); δ = ppm.
MS: 258 (43,M⁺), 243(61), 215(100), 197(34), 155(30), 57(21), 43(90).

### b) 6-(1-Methoxy-vinyl)-1,1,2,4,4,7-hexamethyl-tetralin

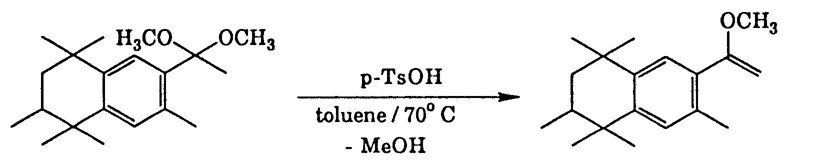

After the distillation of the reaction mass, the product is purified by flash chromatography on silicagel with hexane/diethyl ether 40:1 as eluent. Purity 100% by GC (Supelcowax) mp: 40.2-41.6°C.
¹H-NMR (200 MHz; CDCl₃): 0.98 (d,J=7Hz,3H); 1.04 (s,3H), 1.24 (s,3H); 1.28 (s,3H); 1.30 (s,3H); 1.36 at 1.98 (m,3H); 2.28 (s,3H), 3.68 (s,3H); 4.19 (d,J=2Hz,1H); 4.28 (d,J=2Hz,1H); 7.14 (s,1H); 7.22 (s,1H); δ = ppm.
MS: 272 (52,M⁺), 257(100), 201(17), 183(33), 169(15), 57(18).
IR: 3120, 3020, 2965, 2930, 2880, 1645, 1610, 1270, 1245, 1190, 1120, 1050, 800 cm⁻¹.
Odour: musky, sweet, flowery.

### c) 6-(1-Ethoxy-vinyl)-1,1,2,4,4,7-hexamethyl-tetralin

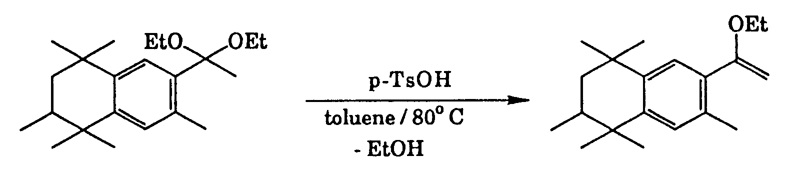

The crude oil is fractionally distilled to yield the pure enol ether (60% yield) purity 98% by GC (Supelcowax), bp: 123°C (1 mbar), solid white product, mp: 43,2-44,8°C.
¹H-NMR (200 MHz; CDCl₃): 0.96 (d,J=7Hz,3H); 1.05 (s,3H); 1.24 (s,3H); 1.29 (s,3H); 1.31 (s,3H); 1.36 (t,J=7Hz,3H); 1.42 at 1.96 (m,3H); 2.31 (s,3H); 3.87 (q,J=7Hz,2H); 4.17 (d,J=2Hz,1H); 4.25 (d,J=2Hz,1H); 7.13 (s,1H); 7.24 (s,1H); δ ppm.
MS: 286 (56,M⁺), 271(80), 257(29), 243(39), 227(100), 201(28), 183(41), 161(34), 57(24), 43(78).
IR: 3120, 3020, 2965, 2930, 2880, 1640, 1600, 1245, 1120, 1060, 800 cm⁻¹.
Odour: fruity, sweet, musky, woody.

### d) 4-(1-Ethoxy-vinyl)-1,1-dimethyl-6-tert-butyl-indan

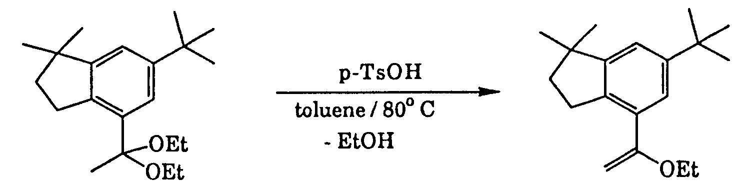

The pure product (95% by GC: Supelcowax) is obtained by distillation under vacuum; Colorless liquid: bp 93°C (0.8 mbar).
¹H-NMR (200 MHz; CDCl₃): 1.25 (s,6H); 1.33 (s,9H); 1.40 (t,J=7Hz,3H); 1.88 (t,J=7Hz,2H), 2.95 (t,J=7Hz,2H); 3.88 (q,J=7Hz,2H); 4.25 (d,J=2Hz,1H); 4.32 (d,J=2Hz,1H); 7.13 (d,J=2Hz,1H); 7.33 (d,J=2Hz,1H); δ = ppm.
MS: 272 (46,M⁺), 257(20), 243(42), 229(30), 213(20), 187(48), 57(54), 43(100).
IR: (pur) 3120, 3060, 2950, 2860, 1645, 1610, 1270, 1245, 1060, 800 cm⁻¹.
Odour: musky, fruity, woody.

### e) 5-(1-Methoxy-vinyl)-1,1,2,3,3,6-hexamethyl-indan

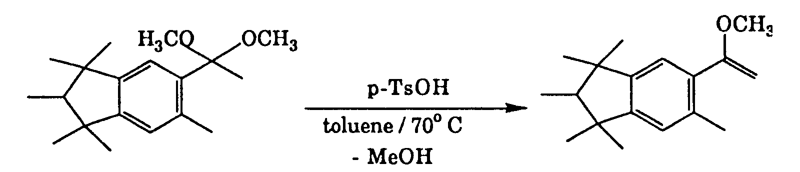

The enol ether is separated from the crude reaction mixture by distillation under vacuum (105°C/2 mbar) and is purified by flash chromatography on silicagel with a mixture hexane/diethylether 50:1 as eluent. Purity 98% by GC (Supelcowax). White solid mp 27.9-31.4°C.
¹H-NMR (200 MHz; CDCl₃): 0.98 (d,J=7Hz,3H); 1.05 (s,6H); 1.26 (s,6H); 1.84 (q,J=7Hz,1H); 2.32 (s,3H); 3.69 (s,3H); 4.20 (d,J=2Hz,1H); 4.30 (d,J=2Hz,1H); 6.95 (s,1H); 7.09 (s,1H); δ = ppm.
MS: 258 (44,M⁺), 243(100), 211(30), 188(21), 169(12), 57(12), 43(8).
IR: (pur) 3120, 3010, 2960, 2865, 1650, 1610, 1270, 1280, 1130, 1050, 800 cm⁻¹.
Odour: musky, slightly fruity.

### f) 5-(1-Ethoxy-vinyl)-1,1,2,3,3,6-hexamethyl-indan

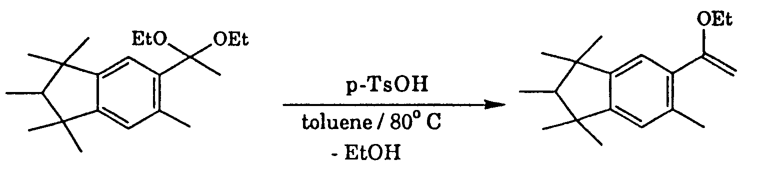

A flash chromatography on silicagel with hexane/diethyl ether 50:1 as eluent of the distilled enol ether (110°C/2 mbar) affords a pure liquid product.

Purity 93% by GC (Supelcowax). Colorless liquid, bp: 110°C (2 mbar).
¹H-NMR (200 MHz; CDCl₃): 0.98 (d,J=7Hz,3H); 1.05 (s,6H); 1.24 (s,6H); 1.36 (q,J=7Hz,3H); 1.84 (q,J=7Hz,1H); 2.33 (s,3H); 3.88 (q,J=7Hz,2H); 4.17 (d,J=2Hz,1H); 4.27 (d,J=2Hz,1H); 6.95 (s,1H); 7.09 (s,1H); δ = ppm.
MS: 272 (56,M⁺), 257(89), 243(37), 229(24), 213(100), 202(72), 57(16), 43(49), 29(18).
IR: (pur) 3120, 3000, 2960, 2870, 1640, 1600, 1285, 1270, 1120, 1110, 1060, 800 cm⁻¹.
Odour: musky, slightly fruity.

### Example 2

### Preparation of single phase liquid mixtures of enol ethers I and carbonyl compounds III

As pointed out before the liquid mixtures enol ethers and carbonyl compounds can be directly prepared by reacting ketones and ortho formic esters (e.g. ethyl or methyl ester) in acidic medium without isolating, i.e. extracting the intermediate ketals.

It is sufficient to adjust the dosage of the starting materials to the desired mixture carbonyl/ether and to remove the formic ester (e.g. ethyl or methyl) and the alcohol (e.g. MeOH or EtOH) formed during the partial acetalyzation and then during the elimination reaction (one mole of alcohol from the ketal). The approriate liquid mixtures are then conveniently flash distilled under vacuum.

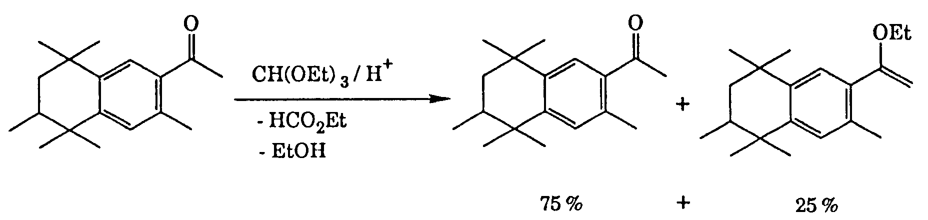

0.4 ml of acetyl chloride are added dropwise within approximately 5 minutes to a mixture of 6 g (0.04 mol) of orthoformic ethyl ester, 4 ml of EtOH and 25.8 g of 6-acetyl-1,1,2,4,4,7-hexamethyl-tetralin (0.1 mol). The reaction mixture is heated at 40°C for 15 minutes until about 30% of acetal is formed. The reaction is monitored by GC. The reaction mass is then heated at 110°C and the ethyl formate and ethanol formed are distilled off. Alter the alcohol has been eliminated, the reaction mass is neutralized by adding a 10% solution of potassium hydroxide in ethanol. A flash distillation under reduced pressure (125°C/2 mbar) gives 22 g of a colorless liquid mixture containing 25% (GC) of 6-(1-ethoxy-vinyl)-1,1,2,4,4,7-hexamethyl-tetralin and 75% of 6-acetyl-1,1,2,4,4,7-hexamethyl-tetralin.
¹H-NMR (200 MHz; CDCl₃): 2.31 (s,0.66H); 2.48 (s,2.35H); 2.57 (s,2.32H); 3.87 (q,J=7Hz,0.36H); 4.17 (d,J=2Hz,0.22H); 4.25 (d,J=2Hz,0.22H); 7.13 (s,0.22H); 7.20 (s,0.74H); 7.24 (s,0.22H); 7.66 (s,0.73H).
Odour: musky, fruity.

### Example 3

### a) Composition for cosmetic consumer goods

| | Parts per weight |
|---|---|
| Compound of Example 1c) | 150.00 |
| Benzyl acetate | 70.00 |
| Vetivenyl acetate | 40.00 |
| Phenyl ethyl alcohol | 150.00 |
| Hexyl cinnamic aldehyde | 100.00 |
| C 10 aldehyde | 2.00 |
| C 11 aldehyde | 1.00 |
| Methyl anthranilate | 1.00 |
| Bergamotte essence | 150.00 |
| Eugenol | 15.00 |
| Gardenol (methyl phenyl carbinyl acetate) | 5.00 |
| Indolene (8,8^{bis}-(3H-indol-3-yl)-2,6-dimethyl-2-octanol) | 2.00 |
| Isoeugenol | 3.00 |
| Isoraldeine 70 (mixture of alpha- and beta-methylionone) | 40.00 |
| Linalool | 50.00 |
| Methyl cedryl cetone | 80.00 |
| Musk ketone (5-t-butyl-1,2,3-trimethyl-4,6-dinitrobenzene) | 10.00 |
| Nonadyl (6,8-dimethyl-2-nonanol) | 30.00 |
| Peche pure (γ-undecalactone) | 1.00 |
| Benzyl salicylate | 50.00 |
| 3-cis-Hexenyl salicylate | 30.00 |
| Sandalore (3-methyl-5-(2,2,3-trimethylcyclopent-3-en-1-yl)pentan-2-ol) | 20.00 |
| | Total: 1000.00 |

The addition of the novel enol ether (a powerful fixative) exalts the warm musky and animalic notes and brings a velvety aspect to the composition. The composition receives much more volume.

### b) Eau de toilette for women

| | Parts per weight |
|---|---|
| Compound of Example 1c) | 150.00 |
| Benzyl acetate | 80.00 |
| Phenyl ethyl alcohol | 120.00 |
| Hexyl cinnamic aldehyde | 160.00 |
| C 11 aldehyde 1%/carbitol | 2.00 |
| Phenyl acetic aldehyde | 2.00 |
| Bergamotte essence | 70.00 |
| Cedar wood ess. Virgin. | 50.00 |
| Evernyl (2,4-dimethyl-3,6-dihydroxy dimethyl benzoate) | 3.00 |
| Geraniol pur | 60.00 |
| Clove bud oil | 8.00 |
| Hedione (methyldihydrojasmonate) | 80.00 |
| Heliotropine crist. | 5.00 |
| Hydroxycitronellal | 120.00 |
| Isoraldeine 95 (mixture of alpha- and beta-methylionone) | 30.00 |
| Mandarine ess. | 15.00 |
| Benzyl salicylate | 50.00 |
| 3-cis Hexenyle salicylate | 10.00 |
| Tropional (trans-3-(3,4-methylendioxyphenyl)-2-methylpropanol) | 15.00 |
| | Total: 1000.00 |

The addition of the novel enol ether confers to the composition an elegant musky note accompanied by a warm and sweet red fruit-like undertone. This addition leads to a round, well balanced and rich Eau de toilette.

The novel enol ether is a good fixative, it brings about volume and richness, two aspects which are appreciated not only in the top notes but throughout the entire evaporation of the resulting perfume.

## Claims

1. Compounds of the general formula 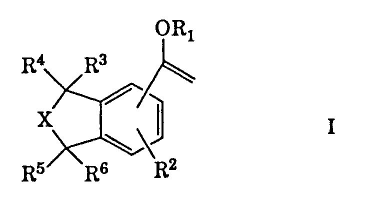 wherein
R¹ is C₁₋₄ alkyl or C₂-₄ alkenyl
R² signifies H or C₁₋₄ alkyl
R³, R⁴, R⁵, R⁶ signify H, CH₃, C₂H₅, CH₂-CH₂-CH₃ or CH(CH₃)₂,
X is methylene, ethylidene, propylidene, ethylene, propylene, isopropylidene or 1,2-dimethylethylene,
and the total number of carbon atoms of R³, R⁴, R⁵, R⁶ is ≤ 6.

2. 6-(1-Ethoxy-vinyl)-1, 1, 4, 4-tetramethyl-tetralin.

3. 6-(1-Methoxy-vinyl)-1, 1, 2, 4, 4, 7-hexamethyl-tetralin.

4. 6-(1-Ethoxy-vinyl-)-1, 1, 2, 4, 4, 7-hexamethyl-tetralin.

5. 4-(1-Ethoxy-vinyl)-1, 1-dimethyl-6-tert-butyl-indan.

6. 5-(1-Methoxy-vinyl)-1, 1, 2, 3, 3, 6-hexamethyl-indan.

7. 5-(1-Ethoxy-vinyl)-1, 1, 2, 3, 3, 6-hexamethyl-indan.

8. Process for the manufacture of the compounds I of Claim 1, comprising removing from a compound of formula 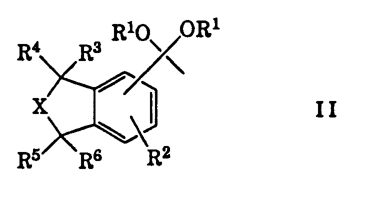 wherein
R¹, R², R³, R⁴, R⁵ and R⁶ have the significance given in claim 1,
one equivalent of the alcohol R¹OH by an acid catalyzed reaction at temperatures between 40°C and 120°C.

9. Process according to Claim 8, wherein the compound II is prepared in situ, preferably from the corresponding ketone.

10. Odorant composition, containing a compound I of Claim 1.

11. The use of the compounds I of Claim 1 as odorants.

## Patentansprüche

1. Verbindungen der allgemeinen Formel 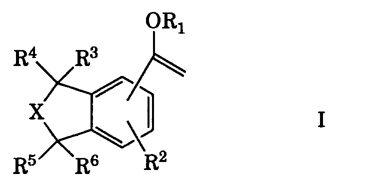 worin
R¹ C₁₋₄ Alkyl oder C₂₋₄ Alkenyl ist,
R² H oder C₁₋₄ Alkyl, und
R³, R⁴, R⁵, R⁶ H, CH₃, C₂H₅, CH₂-CH₂-CH₃ oder CH(CH₃)₂ darstellen, und
X Methylen, Ethyliden, Propyliden, Ethylen, Propylen, Isopropyliden oder 1,2-Dimethyl-ethylen ist,
und die gesamte Anzahl der Kohlenstoffatome von R³, R⁴, R⁵ und R⁶ ≤ 6 ist.

2. 6-(1-Ethoxy-vinyl)-1, 1,4, 4-tetramethyl-tetralin.

3. 6-(1-Methoxy-vinyl)-1, 1, 2, 4, 4, 7-hexamethyl-tetralin.

4. 6-(1-Ethoxy-vinyl-)-1, 1, 2, 4, 4, 7-hexamethyl-tetralin.

5. 4-(1-Ethoxy-vinyl)-1, 1-dimethyl-6-tert-butyl-indan.

6. 5-(1-Methoxy-vinyl)-1, 1, 2, 3, 3, 6-hexamethyl-indan.

7. 5-(1-Ethoxy-vinyl)-1, 1, 2, 3, 3, 6-hexamethyl-indan.

8. Verfahren zur Herstellung der vom Anspruch 1 umfassten Verbindungen I,
dadurch gekennzeichnet, dass aus einer Verbindung der Formel 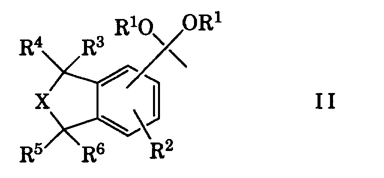 worin
R¹, R², R³, R⁴, R⁵ and R⁶ die in Anspruch 1 angegebene Bedeutung haben,
ein Äquivalent des Alkohols R¹OH durch eine säure-katalisierte Reaktion bei Temperaturen zwischen 40° und 120° C entfernt wird.

9. Verfahren gemäss Anspruch 8, worin die Verbindung II in situ, vorzugsweise aus dem entsprechenden Keton, hergestellt wird.

10. Eine Riechstoffkomposition, die eine im Anspruch 1 definierte Verbindung enthält.

11. Verwendung der in Anspruch 1 definierten Verbindungen I als Riechstoffe.

## Revendications

1. Composés de formule générale 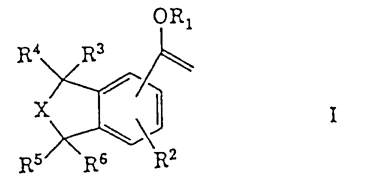 dans laquelle
R¹ est un groupe alkyle en C₁-C₄ ou alcényle en C₂-C₄
R² représente H ou un groupe alkyle en C₁-C₄
R³, R⁴, R⁵, R⁶ représentent H, CH₃, C₂H₅, CH₂CH₂CH₃ ou CH(CH₃)₂,
X est un groupe méthylène, éthylidène, propylidène, éthylène, propylène, isopropylidène ou 1,2-diméthyléthylène,
et le nombre total d'atomes de carbone de R³, R⁴, R⁵, R⁶ est inférieur ou égal à 6.

2. 6-(1-Ethoxyvinyl)-1,1,4,4-tétraméthyltétraline.

3. 6-(1-Méthoxyvinyl)-1,1,2,4,4,7-hexaméthyltétraline.

4. 6-(1-Ethoxyvinyl)-1,1,2,4,4,7-hexaméthyltétraline.

5. 4-(1-Ethoxyvinyl)-1,1-diméthyl-6-t-butylindane.

6. 5-(1-Méthoxyvinyl)-1,1,2,3,3,6-hexaméthylindane.

7. 5-(1-Ethoxyvinyl)-1,1,2,3,3,6-hexaméthylindane.

8. Procédé de fabrication des composés I de la revendication 1, comprenant l'élimination, d'un composé de formule 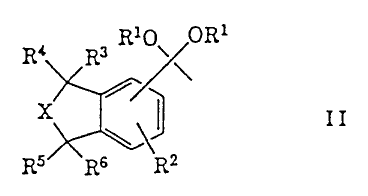 dans laquelle
R¹, R², R³, R⁴, R⁵ et R⁶ ont les significations indiquées dans la revendication 1,
d'un équivalent de l'alcool R¹OH, par une réaction catalysée par un acide, à des températures entre 40°C et 120°C.

9. Procédé selon la revendication 8, dans lequel le composé II est préparé in situ, de préférence à partir de la cétone correspondante.

10. Composition odorante, contenant un composé I de la revendication 1.

11. Utilisation des composés I de la revendication 1 comme substances odorantes.
